(19) Europäisches Patentamt / European Patent Office / Office européen des brevets

(11) **EP 3 576 617 B1**

(12) **EUROPEAN PATENT SPECIFICATION**

(45) Date of publication and mention
of the grant of the patent:
**22.04.2026  Bulletin 2026/17**

(21) Application number: **18748015.7**

(22) Date of filing: **02.02.2018**

(51) International Patent Classification (IPC):
**A61B 5/029** (2006.01)     **A61B 5/021** (2006.01)
**A61B 5/00** (2006.01)

(52) Cooperative Patent Classification (CPC):
**A61B 5/02028; A61B 5/02156; A61B 5/028;**
**A61B 5/029;** A61B 5/0004; A61B 5/7221;
A61B 5/74; A61B 2560/0223

(86) International application number:
**PCT/US2018/016659**

(87) International publication number:
**WO 2018/144875 (09.08.2018 Gazette 2018/32)**

(54) **HEMODYNAMIC MONITOR PROVIDING ENHANCED CARDIAC OUTPUT MEASUREMENTS**

HÄMODYNAMISCHER MONITOR MIT VERBESSERTEN MESSUNGEN DES
HERZZEITVOLUMENS

DISPOSITIF DE SURVEILLANCE HÉMODYNAMIQUE FOURNISSANT DES MESURES DE DÉBIT
CARDIAQUE AMÉLIORÉES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **02.02.2017  US 201762453754 P**
**31.01.2018  US 201815885232**

(43) Date of publication of application:
**11.12.2019  Bulletin 2019/50**

(73) Proprietor: **Becton, Dickinson And Company**
**Franklin Lakes, New Jersey 07417 (US)**

(72) Inventor: **HOLLAND, Alexander**
**Irvine**
**CA 92614**
**(US)**

(74) Representative: **Eisenführ Speiser**
**Patentanwälte Rechtsanwälte PartGmbB**
**Postfach 31 02 60**
**80102 München (DE)**

(56) References cited:
EP-A1- 1 604 705     EP-A2- 1 304 074
EP-B1- 2 506 760     WO-A1-01/03580
US-A- 5 873 834      US-A1- 2002 022 785
US-A1- 2007 197 921    US-A1- 2008 287 812
US-A1- 2013 085 357    US-A1- 2014 303 509

## EP 3 576 617 B1

**Description**

### TECHNICAL FIELD

The subject matter described herein relates to hemodynamic monitors utilizing two or more different physiological sensors to provide enhanced cardiac output measurements.

### BACKGROUND

Cardiac output (CO) provides an indication of the volume of blood being pumped by the heart of a patient at any given time. There are numerous techniques for determining cardiac output, including the arterial pressure cardiac output (APCO) algorithm, and the continuous cardiac output (CCO) and injectate cardiac output (ICO) thermal dilution style algorithms. APCO style algorithms generally have significantly higher bandwidth than CCO or ICO algorithms. Given such an arrangement, APCO algorithm measurements are less averaged or collected more regularly, and hence can better follow rapid or transitory changes in patient cardiac output CO. However, APCO algorithms with peripheral pressure signals as input are in general less accurate than CCO or ICO algorithms that directly measure central blood flow.

US 2008/0287812 A1 discloses methods and systems for estimating cardiac output and total peripheral resistance, which include observing arterial blood pressure waveforms to determine intra-beat and inter-beat variability in arterial blood pressure and estimating from the variability a time constant for a lumped parameter beat-to-beat averaged Windkessel model of the arterial tree. Uncalibrated cardiac output and uncalibrated total peripheral resistance may then be calculated from the time constant. Calibrated cardiac output and calibrated total peripheral resistance may be computed using calibration data, assuming an arterial compliance that is either constant or dependent on mean arterial blood pressure. The parameters of the arterial compliance may be estimated in a least-squares manner.

WO 01/03580 A1 concerns a method for continuous monitoring of cardiac output, in which method cardiac output is measured by a continuous impedance measurement method. In the method, the cardiac output value (SV) obtained via the impedance measurement is calibrated by a quantitative second measuring method.

### SUMMARY

In one aspect, first data is continuously received that is generated by a first physiological sensor measuring at least one physiological parameter of a patient. In addition, second data is continuously received that is generated by a second physiological sensor concurrently measuring the at least one physiological parameter of the patient. The first physiological sensor measures the at least one physiological parameter at a higher bandwidth with lower precision as compared to the second physiological sensor. The continuously received first data is adaptively calibrated using the continuously received data to result in a continually updating calibrated measurement. Data characterizing the continually updating calibrated measurement can then be provided.

The providing data can take many forms including, for example, one or more of: displaying the data characterizing the calibrated measurement in an electronic visual display, transmitting the data characterizing the calibrated measurement to a remote computing system, loading the data characterizing the calibrated measurement into memory, and/or storing the data characterizing the calibrated measurement in physical data persistence.

The at least one physiological parameter is cardiac output.

The first physiological sensor is used to measure arterial pressure cardiac output. The first physiological sensor can include a cuff to be placed on an extremity of the patient and utilizing a volume clamp method to calculate one or more of: stroke volume, stroke volume variation, APCO, systemic vascular resistance (SVR), and/or continuous blood pressure (cBP).

The second physiological sensor is used to measure continuous cardiac output and/or injectate cardiac output. The second physiological sensor can include a pulmonary artery catheter (PAC) that is inserted into a pulmonary artery of the patient to detect cardiac pressures in the patient by way of a thermal filament located on the catheter. The second physiological sensor can additionally or alternatively measure cardiac output using a bolus thermodilution method.

The adaptive calibration can be based on a time-varying linear scaling and an offset calculated using a least mean-square error solution. Measurement values within the first data can be time averaged over a time window length corresponding to a periodicity of measurements of the second physiological sensor. The time averaged measurement values can be weighted based on a standard deviation of the measurements from each of the first physiological sensor and the second physiological sensor. It can be determined, if a measurement value exceeds a pre-defined standard of deviation value, and the measurement can be characterized as good if it does not exceeds the pre-defined standard of deviation value and the measurement can be characterized as bad if it exceeds the pre-defined standard of deviation value.

The time averaged measurement values can be weighted based on a forgetting factor.

2

Non-transitory computer program products (i.e., physically embodied computer program products) are also described that store instructions, which when executed by one or more data processors of one or more computing systems, cause at least one data processor to perform operations herein.

Similarly, computer systems are also described that can include one or more data processors and memory coupled to the one or more data processors. The memory can temporarily or permanently store instructions that cause at least one processor to perform one or more of the operations described herein. Such systems can include one or more of the first physiological sensor and the second physiological sensor.

In addition, methods can be implemented by one or more data processors either within a single computing system or distributed among two or more computing systems. Such computing systems can be connected and can exchange data and/or commands or other instructions or the like via one or more connections, including but not limited to a connection over a network (e.g., the Internet, a wireless wide area network, a local area network, a wide area network, a wired network, or the like), via a direct connection between one or more of the multiple computing systems, etc.

The subject matter described herein provides many technical advantages. For example, the current subject matter can provide enhanced physiological measurements by calibrating the output of a first physiological sensor having a greater bandwidth with lower precision using the output of a second physiological sensor having a lower bandwidth with higher precision. In particular, in some implementations, the current subject matter provides enhanced APCO measurements that provide accuracy similar to CCO or ICO measurements while also tracking cardiac output variations that can only be detected using a higher bandwidth.

The details of one or more variations of the subject matter described herein are set forth in the accompanying drawings and the description below. Other features and advantages of the subject matter described herein will be apparent from the description and drawings, and from the claims.

## DESCRIPTION OF DRAWINGS

FIG. 1 is a logical diagram illustrating a hemodynamic monitor in communication with two physiological sensors affixed to a patient;

FIG. 2 is a simulated measurements diagram illustrating arterial pressure cardiac output, continuous cardiac output, adaptively and linearly calibrated arterial cardiac output, and true cardiac output in relation to one another;

FIG. 3A is a first diagram of real measurements illustrating arterial pressure cardiac output, continuous cardiac output, and adaptively and linearly calibrated arterial cardiac output, in relation to one another;

FIG. 3B is a second diagram of real measurements illustrating arterial pressure cardiac output, continuous cardiac output, and adaptively and linearly calibrated arterial cardiac output, in relation to one another;

FIG. 4 is a diagram illustrating calibration of a hemodynamic measurement by a hemodynamic monitor such as in FIG. 1; and

FIG. 5 is a diagram illustrating a computing device for implementing aspects of the current subject matter.

## DETAILED DESCRIPTION

The current subject matter is directed to systems, methods, and articles that use a lower bandwidth physiological sensor measurement (i.e., a measurement that is taken less frequently) to calibrate a higher bandwidth physiological measurement (i.e., a measurement that is take more frequently than the lower bandwidth physiological sensor measurement) to provide more precise characterization of the condition of a patient (which in turn results in improved patient care). While the current subject matter provides examples for the calculation of cardiac output (CO), unless otherwise specified, the current subject matter can be applicable to other types of physiological sensors in which different underlying measurement techniques are utilized to measure a same or similar physiological condition and one such technique has lower bandwidth / resolution as compared to one or more other techniques. Such measurements can relate to both hemodynamic as well as non-hemodynamic physiological measurements.

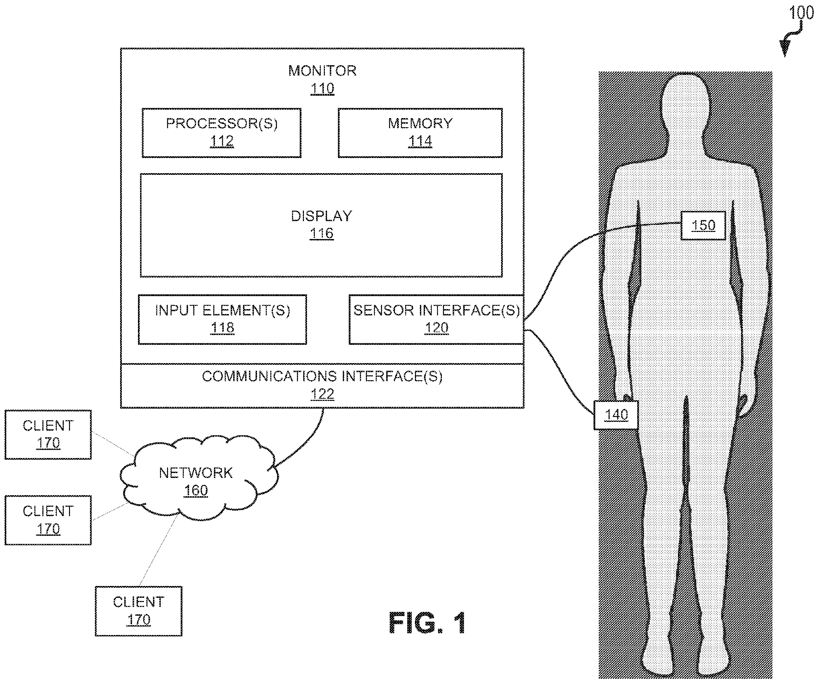

FIG. 1 is a diagram 100 in which a hemodynamic (HD) monitor 110 is configured to receive data characterizing various measured physiological parameters of a patient 130 from a first physiological sensor 140 and a second physiological sensor 150. The HD monitor 110 includes at least one programmable data processor 112 (which may have multiple processing cores), memory 114 for storing instructions for execution by the at least one programmable processor 112, an electronic visual display 116 for rendering a graphical user interface for displaying information that characterizes the measured physiological parameters. Such information can take various forms including waveforms, numerical indications, categorical indications, and the like. The HD monitor 110 can also include various interface input elements 118 which can be physically manipulated to affect operation of the HD monitor 110 such as what information is being displayed on the display 116 and/or configuration information for the first physiological sensor 140 and/or the second physiological sensor 150. In addition or in the alternative, the display 116 can comprise a touch-screen interface allowing users to select graphical user interface elements directly.

The HD monitor 110 can further include a sensor interface 120 that enables data to be received from and optionally additionally transmitted to one or more physiological sensors including the first physiological sensor 140 and the second physiological sensor 150. The sensor interface 120 can communicate with the first physiological sensor 140 and/or the second physiological sensor 150 using a physical wired connection and/or using a wireless data protocol. The HD monitor 110 can also include at least one communications interface 122 that can enable direct or indirect communication with one or more remote client computing systems 170 via a wired and/or wireless network 160. For example, the HD monitor 110 can convey / exchange data with a remote computing system (e.g., the HD monitor 110 can transmit the physiological measurements for storage by a remote database / cloud-storage service, the HD monitor 110 can receive contextual / historical information about the patient which can be used herein, etc.).

The first physiological sensor 140 can be or include a peripheral artery pressure sensor or one or more finger cuffs that can be used to calculate various hemodynamic parameters including stroke volume, stroke volume variation, APCO, systemic vascular resistance (SVR) and continuous blood pressure (cBP). The finger cuffs can perform real-time finger pressure measurements using a volume clamp method at a sampling rate of, for example, 1000 times per second.

The second physiological sensor 150 can be or include a pulmonary artery catheter (PAC) such as a Swan-Ganz catheter. Such catheter can be inserted into a pulmonary artery of the patient 130 to detect direct, simultaneous measurement of pressures in the right atrium, right ventricle, pulmonary artery, and the filling pressure of the left atrium of the patient 130 by way of a thermal filament located on the catheter and using thermodilution principles. In addition or in the alternative, the second physiological sensor 150 can be used to measure CO using bolus thermodilution methods. The second physiological sensor 150 can be used to implement the CCO or ICO algorithms.

The techniques described herein can be processed by the at least one programmable data processor 112 of the HD monitor 110 and/or such processing many be offloaded to one or more of the first physiological sensor 140 or the second physiological sensor 150 or a remote client computing device 170 (which may access the underlying data via the communications interface(s) 122).

With the current arrangement, the CCO or ICO algorithm as provided by the second physiological sensor 150 can act as a calibration for a relatively high bandwidth APCO algorithm as provide by the first physiological sensor 140 in order to estimate the patient's 130 time varying cardiac output, $c(t)$, with accuracy akin to a CCO/ICO algorithm and with bandwidth akin to an APCO algorithm. While the following refers to the second physiological sensor 150 as implementing the CCO algorithm, the thermal dilution input measurements may come from a CCO, an ICO, or a mix of ICO or CCO algorithms. In addition, while the following only refers to CO algorithm output parameter, the current calibration techniques can be applied to other hemodynamic parameter outputs from both algorithms.

One approach employed by the HD monitor 110 can iteratively compute a time-varying linear scaling, $A[n]$, and an offset, $B[n]$, that update an APCO measurement generated by the first physiological sensors 150, $m_F[n]$, as follows

$$\hat{m}[n] = A[n]m_F[n] + B[n] \qquad (1)$$

Parameters $A[n]$ and $B[n]$ can be functions of the past history of CO estimates. These functions can act to adjust the APCO measurement to more closely correlate with a CCO measurement. As used herein $m_F$ can be referred to as "measurement fast", but relatively inaccurate measurement and $m_A$ can refer to as "measurement accurate", but relatively low bandwidth.

Namely, $A[n]$ and $B[n]$ can be a least mean-square error solution to the following linear equations that arise by replacing $\hat{m}[n]$ in eq. 1 with CCO measurements and $m_F[n]$ with averaged APCO measurements,

$$\begin{bmatrix} m_A[0] \\ m_A[1] \\ \vdots \\ m_A[n] \end{bmatrix} = \begin{bmatrix} \overline{m}_F[0] & 1 \\ \overline{m}_F[1] & 1 \\ \vdots \\ \overline{m}_F[n] & 1 \end{bmatrix} \begin{bmatrix} A[n] \\ B[n] \end{bmatrix} \qquad (2)$$

$$\mathbf{m}_A[n] = \begin{bmatrix} \overline{\mathbf{m}}_F[n] & 1 \end{bmatrix} \begin{bmatrix} A[n] \\ B[n] \end{bmatrix}$$

where

$$\mathbf{m}_A[n] = [m_A[0], m_A[1], \ldots, m_A[n-1], m_A[n]]^T \qquad (3)$$

is a sequence of discrete CCO estimates arriving at times,

$$t_A[0], t_A[1], \ldots, t_A[n-1], t_A[n] \qquad (4)$$

and where each time value, $t_A[n]$, represents the average value of $c(t)$ over an immediately preceding window of time, $w_A[n]$, given by the corresponding sequence,

$$w_A[0], w_A[1], \ldots, w_A[n-1], w_A[n] \qquad (5)$$

The sequence of APCO estimates,

$$\overline{\mathbf{m}}_F[n] = [\overline{m}_F[n], \overline{m}_F[n-1], \ldots, \overline{m}_F[1], \overline{m}_F[0]]^T \qquad (6)$$

can represent averages over a time period that is as close as possible to the averaging time window corresponding to $m_A[n]$. An APCO measurement with a line over it, $m_F[k]$, signifies the averaging time adjustment for $m_F[k]$.

An optimum least squares solution for $[A[n]B[n]]^T$ can minimize the weighted squared error,

$$\min_{A,B} \epsilon^2 = \min_{A,B} \left\| \mathbf{m}_A - [\overline{\mathbf{m}}_F \quad 1] \begin{bmatrix} A \\ B \end{bmatrix} \right\|_\Omega^2 = \qquad (7)$$

$$\min_{A,B} \left[ \mathbf{m}_A^T - [A \quad B] \begin{bmatrix} \overline{\mathbf{m}}_F^T \\ 1^T \end{bmatrix} \right] \Omega \left[ \mathbf{m}_A - [\overline{\mathbf{m}}_F \quad 1] \begin{bmatrix} A \\ B \end{bmatrix} \right]$$

where for clarity, the dependency on $n$ is not shown. The positive definite matrix,

$$\Omega = \begin{bmatrix} \omega[0] & 0 & \ldots & 0 \\ 0 & \omega[1] & \ddots & \vdots \\ \vdots & \ddots & \ddots & 0 \\ 0 & \ldots & 0 & \omega[n] \end{bmatrix} \qquad (8)$$

weights by $\omega[n]$ the relative information of the $n^{th}$ measurement. Measurements that are known to be more accurate provide more information and could be weighted higher, while those that are less accurate provide less information and could be weighted lower. For example, if one had the standard deviations for each accurate and averaged fast measurement, $\sigma_A[n] + \sigma_F[n]$, a reasonable choice for each weight could be

$$\omega[n] = \frac{1}{\sigma_A[n] + \sigma_F[n]} \qquad (9)$$

However, if one could classify which measurements were "good" and which were "bad", another option for the weighting parameters could be

$$\omega[n] = \begin{cases} 1 & \text{if measurement is good} \\ 0 & \text{if measurement is bad} \end{cases} \qquad (10)$$

The important point is that any measure of relative measurement information could be used for the weighting. Weighting can be ignored by setting all weights the same, e.g. $\omega[n] = 1$.

For computing the average, the HD monitor 110 can, for example, save current and past values of the APCO measurements,

$$\mathbf{m_F}[k] = [m_F[k], m_F[k-1], \ldots, m_F[k-N+1]]^T \qquad (11)$$

with corresponding sample times,

$$\mathbf{t_F}[k] = [t_F[k], t_F[k-1], t_F[k-2], \ldots, t_F[k-N+1]]^T \qquad (12)$$

and with corresponding averaging window lengths, $\omega_F[k]$,

$$\mathbf{w_F}[k] = [w_F[k], w_F[k-1], w_F[k-2], \ldots, w_F[k-N+1]]^T \qquad (13)$$

Measurement $m_F[k]$ corresponds to time $i\omega[k] -_F[k]/2$. $N$ is sufficiently large to cover the worst case delay between a CCO measurement and an APCO measurement $\omega$, plus the worst case CCO averaging time window, $_A[n]$. Generally, the averaging window lengths for an APCO algorithm are constant.

The discrete time index, k, for APCO estimates is different from index $n$ for CCO estimates, because in general APCO and CCO algorithm estimates are (a) not synchronized to a common sampling clock, (b) do not have the same sample time intervals, and (c) nor do they have simple integer or rational fraction related sample time intervals.

Multiplying both sides of eq. 2 by $\Omega$ and transposing the right hand side matrix results in the following equivalent linear equations,

$$\begin{bmatrix} S_{FA}[n] \\ S_A[n] \end{bmatrix} = \begin{bmatrix} S_{FF}[n] & S_F[n] \\ S_F[n] & M[n] \end{bmatrix} \begin{bmatrix} A[n] \\ B[n] \end{bmatrix} \qquad (14)$$

where

$$S_F[n] = \sum_{k=0}^{n} \omega^2[k]\overline{m}_F[k] \qquad (15)$$

$$S_{FF}[n] = \sum_{k=0}^{n} \omega^2[k]\overline{m}_F^2[k] \qquad (16)$$

$$S_{FA}[n] = \sum_{k=0}^{n} \omega^2[k]\overline{m}_F[k]m_A[k] \qquad (17)$$

$$S_A[n] = \sum_{k=0}^{n} \omega^2[k]m_A[k] \qquad (18)$$

$$M[n] = \sum_{k=0}^{n} \omega^2[k] \qquad (19)$$

All key parameters used to solve for (adapt) *A[n]* and *B[n]* can be updated iteratively for new CCO, $m_A[n+1]$, and averaged APCO, $m_F[n+1]$, measurements as follows,

$$S_F[n+1] \;=\; S_F[n] + \omega^2[n+1]\overline{m}_F[n+1] \tag{20}$$

$$S_{FF}[n+1] \;=\; S_{FF}[n] + \omega^2[n+1]\overline{m}_F^2[n+1] \tag{21}$$

$$S_{FA}[n+1] \;=\; S_{FA}[n] + \omega^2[n+1]\overline{m}_F[n+1]m_A[n+1] \tag{22}$$

$$S_A[n+1] \;=\; S_A[n] + \omega^2[n+1]m_A[n+1] \tag{23}$$

$$M[n+1] \;=\; M[n] + \omega^2[n+1] \tag{24}$$

However, a problem with these iterations is that measurements far in the past have the same contribution to the solution as more recent measurements. In fact, averaging that includes increasingly larger numbers of measurements, implies that more recent measurements will have less and less overall impact on the calibration over time.

One fix to this problem discounts past measurements using a "forgetting parameter", $0 < y < 1$,

$$S_F[n+1] \;=\; \gamma S_F[n] + \omega^2[n+1]\overline{m}_F[n+1] \tag{25}$$

$$S_{FF}[n+1] \;=\; \gamma S_{FF}[n] + \omega^2[n+1]\overline{m}_F^2[n+1] \tag{26}$$

$$S_{FA}[n+1] \;=\; \gamma S_{FA}[n] + \omega^2[n+1]\overline{m}_F[n+1]m_A[n+1] \tag{27}$$

$$S_A[n+1] \;=\; \gamma S_A[n] + \omega^2[n+1]m_A[n+1] \tag{28}$$

$$M[n+1] \;=\; \gamma M[n] + \omega^2[n+1] \tag{29}$$

Setting $\gamma$ to a small value or "high forgetting" places more weight on the most recent measurement relative to the past, while setting it to a value near 1 or "low forgetting" places more weight on the past.

A second fix can use a constant number P of the most recent measurements by iteratively subtracting the oldest measurement from the totals,

$$S_F[n+1] \;=\; S_F[n] + \omega^2[n+1]\overline{m}_F[n+1] - \\ \omega^2[n+1-P]\overline{m}_F[n+1-P] \tag{30}$$

$$S_{FF}[n+1] \;=\; S_{FF}[n] + \omega^2[n+1]\overline{m}_F^2[n+1] - \\ \omega^2[n+1-P]\overline{m}_F^2[n+1-P] \tag{31}$$

$$S_{FA}[n+1] \;=\; S_{FA}[n] + \omega^2[n+1]\overline{m}_F[n+1]m_A[n+1] - \\ \omega^2[n+1-P]\overline{m}_F[n+1-P]m_A[n+1-P] \tag{32}$$

$$S_A[n+1] \;=\; S_A[n] + \omega^2[n+1]m_A[n+1] - \qquad (33)$$
$$\omega^2[n+1-P]m_A[n+1-P]$$

$$M[n+1] \;=\; M[n] + \omega^2[n+1] - \omega^2[n+1-P] \qquad (34)$$

In one possible variation to solve linear eq. 14, a solution can be determined by way of inverting the matrix. In particular, inverting the right hand side matrix of eq. 14 yields the following solution for $A[n]$ and $B[n]$,

$$\begin{bmatrix} A[n] \\ B[n] \end{bmatrix} = \frac{\begin{bmatrix} M[n] & -S_F[n] \\ -S_F[n] & S_{FF}[n] \end{bmatrix}}{D[n]} \begin{bmatrix} S_{FA}[n] \\ S_A[n] \end{bmatrix} \qquad (35)$$

$$\begin{bmatrix} A[n] \\ B[n] \end{bmatrix} = \begin{bmatrix} (M[n]S_{FA}[n] - S_F[n]S_A[n])/D[n] \\ (-S_F[n]S_{FA}[n] + S_{FF}[n]S_A[n])/D[n] \end{bmatrix}$$

where determinant

$$D[n] \;=\; M[n]S_{FF}[n] - (S_F[n])^2 \qquad (36)$$

provides a means to assess numerical conditioning. The calibration parameters should not be updated if $D[n]$ is close to 0.

Whether a finite, equally weighted measurement history is utilized by the HD monitor 110, or a forgetting factor iteratively weighted infinite past is utilized, linear eq. 14 and its solution eq. 35 are exactly the same. Only the iterative updates for time varying equation parameters change depending on which iterative update method is being used.

Other linear equation solution methods that can be utilized by the HD monitor 110 include, without limitation, Gaussian elimination, QR factorization, Householder reflectors, and the like. Solution via multiplication by orthogonal matrices ("Householder reflectors"), for example, a numerical stable method that does not amplify errors.

In practice, it is expected that to have lower likelihood for greater differences of scaling parameter $A$ from 1 or for greater differences of offset parameter $B$ from 0. Adding a "regularization" term to the weighted least squares optimization problem of eq. 7 provides a new optimization problem that takes into account the lower likelihood of large A - 1 or $B$,

$$\min_{A,B} \epsilon^2 = \min_{A,B} \left\{ \left\| \mathbf{m}_A - \begin{bmatrix} \overline{\mathbf{m}}_F & 1 \end{bmatrix} \begin{bmatrix} A \\ B \end{bmatrix} \right\|_\Omega^2 + \alpha(A-1)^2 + \beta B^2 \right\} \qquad (37)$$

where for clarity, the dependency on $n$ is not shown. The first term is the same least squares fit as eq. 7. The second term is a parameter constraint that penalizes scaling parameter $A$ being different from 1, where the constant $\alpha > 0$ is a relative weight on the "importance" of this constraint relative to the other terms. The third term is parameter constraint that penalizes the offset parameter $B$ being different from 0. The constant $\beta > 0$ is a relative weight on the importance of this constraint relative to the other terms. For example, if keeping $B$ close to 0 has almost no importance, while keeping $A$ close to 1 has relatively high importance, $\alpha$ =0.9, $\beta$ =0.1 weights the latter 9 times higher than the former. The first term always has a weighting of 1.0, making it more important in this case than the parameter constraints. If $\alpha = \beta = 0$, this becomes equivalent to the original eq. 7 least squares optimization.

Equating the derivative of eq. 37 to zero with respect to $A$ and $B$, assuming either a finite or a forgetting factor weighted measurement history, yields the following linear equations for the regularized least squares optimization,

$$\begin{bmatrix} S_{FA}[n] + \alpha \\ S_A[n] \end{bmatrix} = \begin{bmatrix} S_{FF}[n] + \alpha & S_F[n] \\ S_F[n] & M[n] + \beta \end{bmatrix} \begin{bmatrix} A[n] \\ B[n] \end{bmatrix} \qquad (38)$$

with the following matrix inverse solution,

$$\begin{bmatrix} A[n] \\ B[n] \end{bmatrix} = \frac{\begin{bmatrix} M[n] + \beta & -S_F[n] \\ -S_F[n] & S_{FF}[n] + \alpha \end{bmatrix}}{D[n]} \begin{bmatrix} S_{FA}[n] + \alpha \\ S_A[n] \end{bmatrix} \qquad (39)$$

$$D[n] = (M[n] + \beta)(S_{FF}[n] + \alpha) - (S_F[n])^2$$

$$\begin{bmatrix} A[n] \\ B[n] \end{bmatrix} = \begin{bmatrix} (S_{FA}[n] + \alpha)(M[n] + \beta) - S_F[n]S_A[n])/D[n] \\ (-S_F[n](S_{FA}[n] + \alpha) + (S_{FF}[n] + \alpha)S_A[n])/D[n] \end{bmatrix}$$

The HD monitor 110 can implement the techniques provided herein using a variety of software and/or hardware implementations. In one implementation, a Python module can include a measurement "Combiner class" that implements the adaptive least squares mathematics to combine a fast, but less accurate algorithm measurement with a second algorithm measurement that is slower, but relatively more accurate. The implementation provides an option to use a forgetting factor $\gamma$, or to use a finite number of past accurate algorithm estimates. In the latter case, the number of past measurements is a function of $\gamma$, namely P = 1/(1 - y). Variable names and numerical implementation mnemonically match mathematical notation in this document.

The Combiner class can assume that different fast or accurate measurements may arrive asynchronously at different times. There can be separate interface functions to update the algorithm with these measurements. A first function (e.g., function UpdateFast(c,t,w)) can provide the means to inform the algorithm of a new fast measurement m along with the measurement time, t, and the averaging time window, w. The measurement represents time window *[t-w,t]*. A second function (e.g., UpdateAccurate *(c,t,w))* is an analogous function to inform the algorithm of a new accurate algorithm measurement. A third function (e.g., function Combine()) can return a combined measurement.

If measurement algorithm averaging is uniform or symmetrical over window $\omega_A[k]$ for measurement *m[k]*, the measurement corresponds to the time point, $t_A[k] - \omega_A[k]/2$. If averaging is not uniform or symmetrical, the measurement algorithm implementation should provide an input/output Delay() function to return how far in the past that the current measurement corresponds. In general, it is typically better to use a measurement algorithm Delay() function regardless, in order to keep the measurement combining algorithm independent of the individual fast or accurate measurement algorithm averaging policies.

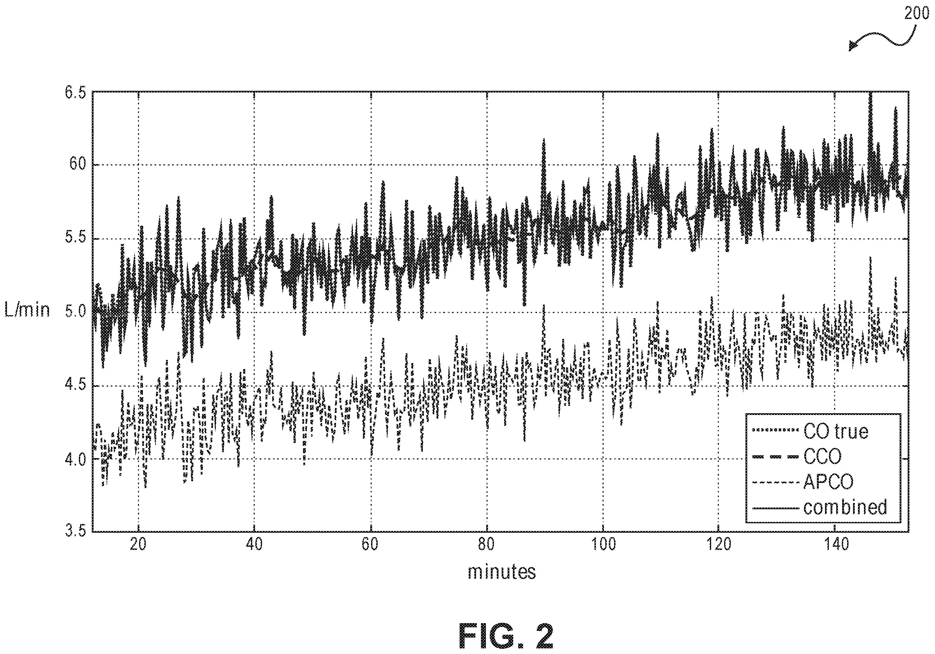

In one simulation, a random "true CO" was generated every 20 seconds, from which the CCO measurements were an average of this sequence over 3 minutes. To simulate less accuracy, the APCO measurements were scaled and an offset adjustment of these measurements was applied. Diagram 200 of FIG. 2 shows a simulation run and Table 1 lists a mean-square error for each of the measurements for several simulation runs. FIG. 2 shows that the combined CO estimate follows both the low bandwidth trend of the CCO measurement and the higher bandwidth variation of the APCO estimate. Table 1 shows that the combined estimate mean-square error is almost an order of magnitude better than the CCO (accurate, but slow algorithm) estimate. In FIG. 2, a first line pattern is a simulated "true CO", a second line pattern is a simulated accurate but averaged CCO, and a third line is an un-averaged, but less accurate APCO, with the fourth line being a combined measurement. The simulation used a finite history *P* = 10 (minutes) of CCO results. Plots can be shifted to account for algorithm input/output delay to align corresponding measurements. Measurement information weighting values $\omega$ *[n]* were random between 0 and 1.

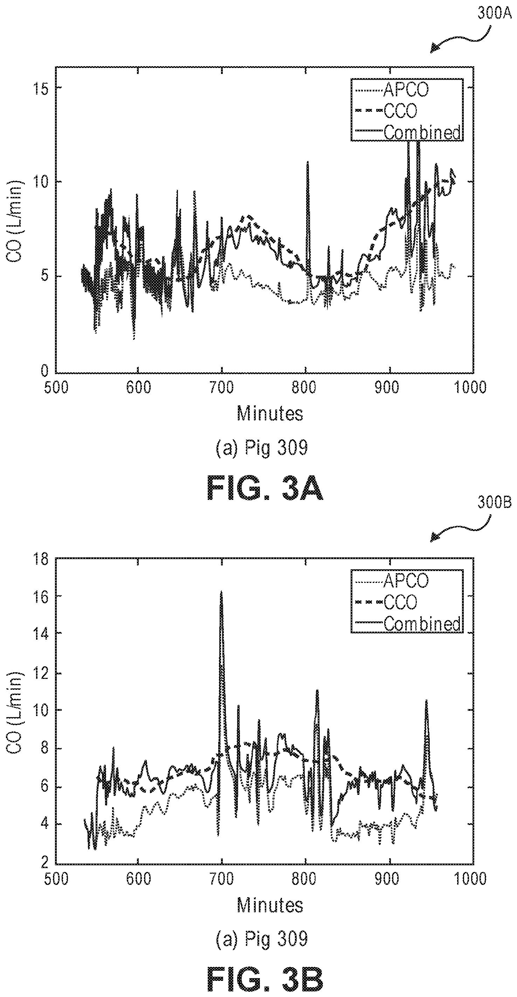

FIGs. 3A and 3B are diagrams 300A, 300B that illustrate operation on challenging porcine temperature and pressure waveforms where vasopressor administration along with induced bleeding, and fluid administration caused intentional significant swings in CO not common in human patients. As can be seen, the combined measurement has similar average (accuracy) of the CCO algorithm along with variable CO swings highly correlated to the swings of the APCO algorithm.

Table 1 below shows a comparison of the mean-square CO estimation errors (L/min) for the three algorithms (APCO, CCO and the combined techniques provided here) using 9600 measurement samples per simulation run. The combined technique used a finite history *P* = 10 (minutes) of CCO results to calibrate A APCO.

TABLE 1

| Run# | APCO | CCO | Combined |
|------|------|------|----------|
| 1 | 1.004 | 0.036 | 0.005 |
| 2 | 1.003 | 0.036 | 0.005 |

(continued)

| Run# | APCO | CCO | Combined |
|---|---|---|---|
| 3 | 1.004 | 0.036 | 0.005 |
| 4 | 1.002 | 0.036 | 0.005 |
| 5 | 1.002 | 0.036 | 0.005 |

FIG. 3 are diagrams 300A and 300 B in which a first line pattern is FloTrac APCO, a second line pattern is a CCO measurement, and a third line pattern is the combined measurement. With forgetting factor y = 0.8, the approximate finite history to fit $P$ = 5 minutes. Regularization parameters $\alpha$ = 1.0 and $\beta$ = 0.5. There was no variable measurement information weighting ($\omega[n]$ = 1).

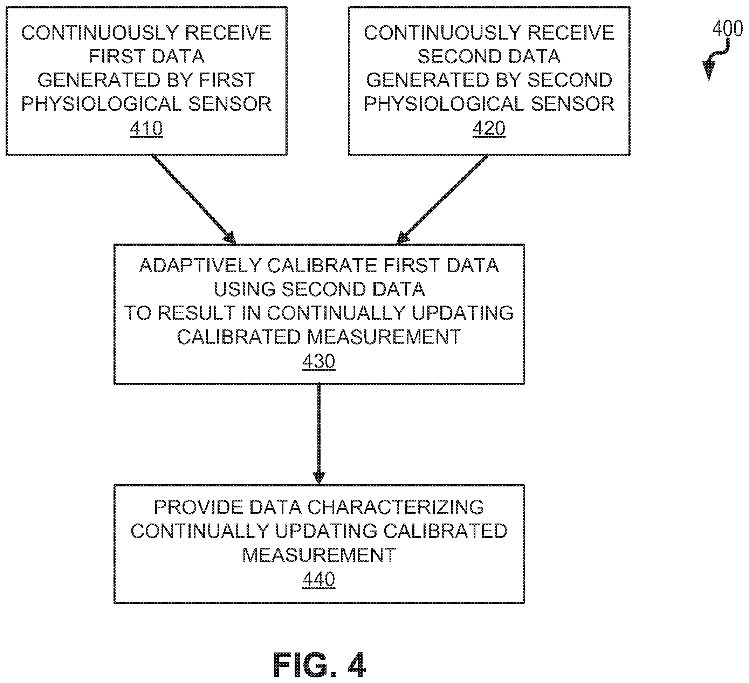

FIG. 4 is a diagram 400 in which, at 410, first data that is generated by a first physiological sensor measuring at least one hemodynamic parameter of a patient is continuously received. In addition, at 420, second data that is generated by a second physiological sensor concurrently measuring the at least one hemodynamic parameter of the patient is continuously received. The first physiological sensor measures the at least one hemodynamic parameter at a higher bandwidth with lower precision as compared to the second physiological sensor. The continuously received first data is adaptively calibrated, at 430, using the continuously received second data to result in a continually updating calibrated measurement. Data characterizing the calibrated measurement is, at 440, provided (e.g., displayed in an electronic visual display, loaded into memory, stored in physical data persistence, and/or transmitted to a remote computing device, etc.).

One or more aspects or features of the subject matter described herein can be realized in digital electronic circuitry, integrated circuitry, specially designed application specific integrated circuits (ASICs), field programmable gate arrays (FPGAs) computer hardware, firmware, software, and/or combinations thereof. These various aspects or features can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device. The programmable system or computing system can include clients and servers. A client and server are generally remote from each other and typically interact through a communication network. The relationship of client and server arises by virtue of computer programs running on the respective computers and having a client-server relationship to each other.

These computer programs, which can also be referred to as programs, software, software applications, applications, components, or code, can include machine instructions for a programmable processor, and/or can be implemented in a high-level procedural language, an object-oriented programming language, a functional programming language, a logical programming language, and/or in assembly/machine language. As used herein, the term "computer-readable medium" refers to any computer program product, apparatus and/or device, such as for example magnetic discs, optical disks, solid-state storage devices, memory, and Programmable Logic Devices (PLDs), used to provide machine instructions and/or data to a programmable data processor, including a machine-readable medium that receives machine instructions as a computer - readable signal. The term "computer-readable signal" refers to any signal used to provide machine instructions and/or data to a programmable data processor. The computer - readable medium can store such machine instructions non-transitorily, such as for example as would a non-transient solid-state memory or a magnetic hard drive or any equivalent storage medium. The computer -readable medium can alternatively or additionally store such machine instructions in a transient manner, such as for example as would a processor cache or other random access memory associated with one or more physical processor cores.

The computer components, software modules, functions, data stores and data structures described herein can be connected directly or indirectly to each other in order to allow the flow of data needed for their operations. It is also noted that a module or processor includes but is not limited to a unit of code that performs a software operation, and can be implemented for example as a subroutine unit of code, or as a software function unit of code, or as an object (as in an object-oriented paradigm), or as an applet, or in a computer script language, or as another type of computer code. The software components and/or functionality can be located on a single computer or distributed across multiple computers depending upon the situation at hand.

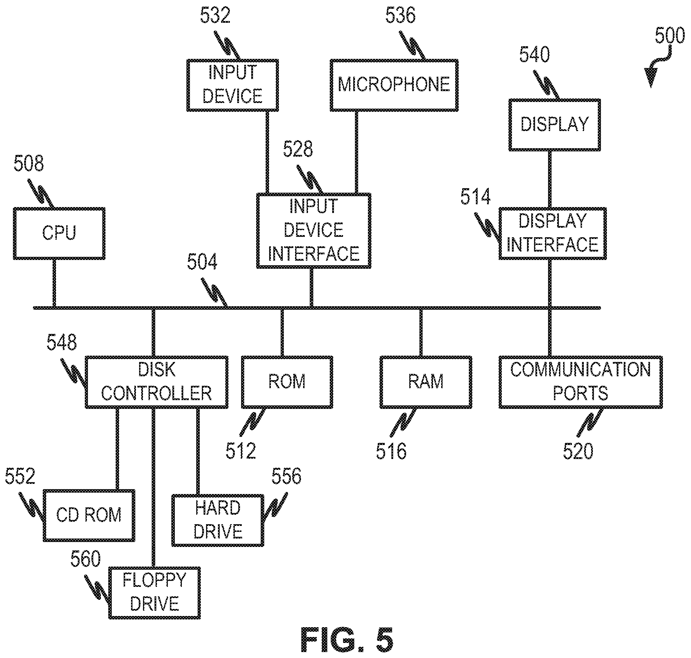

FIG. 5 is a diagram 500 illustrating a sample computing device architecture for implementing various aspects described herein. A bus 504 can serve as the information highway interconnecting the other illustrated components of the hardware. A processing system 508 labeled CPU (central processing unit) *(e.g.,* one or more computer processors / data processors at a given computer or at multiple computers), can perform calculations and logic operations required to execute a program. A non-transitory processor-readable storage medium, such as read only memory (ROM) 512 and random access memory (RAM) 416, can be in communication with the processing system 508 and can include one or more programming instructions for the operations specified here. Optionally, program instructions can be stored on a non-transitory computer-readable storage medium such as a magnetic disk, optical disk, recordable memory device, flash memory, solid-state or other physical storage medium.

In one example, a disk controller 548 can interface one or more optional disk drives to the system bus 504. These disk drives can be external or internal floppy disk drives such as 560, external or internal CD-ROM, CD-R, CD-RW or DVD, or solid state drives such as 552, or external or internal hard drives 556. As indicated previously, these various disk drives 552, 556, 560 and disk controllers are optional devices. The system bus 504 can also include at least one communication port 520 to allow for communication with external devices either physically connected to the computing system or available externally through a wired or wireless network. In some cases, the communication port 520 includes or otherwise comprises a network interface.

To provide for interaction with a user, the subject matter described herein can be implemented on a computing device having a display device 540 (e.g., a CRT (cathode ray tube) or LCD (liquid crystal display) monitor, touchscreen, etc.) for displaying information obtained from the bus 504 to the user and an input device 532 such as keyboard and/or a pointing device (e.g., a mouse or a trackball) and/or a touchscreen by which the user can provide input to the computer. Other kinds of input devices 532 can be used to provide for interaction with a user as well; for example, feedback provided to the user can be any form of sensory feedback (e.g., visual feedback, auditory feedback by way of a microphone 536, or tactile feedback); and input from the user can be received in any form, including acoustic, speech, or tactile input. In the input device 532 and the microphone 536 can be coupled to and convey information via the bus 504 by way of an input device interface 528. Other computing devices, such as dedicated servers, can omit one or more of the display 540 and display interface 524, the input device 532, the microphone 536, and input device interface 528.

In the descriptions above and in the claims, phrases such as "at least one of" or "one or more of" can occur followed by a conjunctive list of elements or features. The term "and/or" can also occur in a list of two or more elements or features. Unless otherwise implicitly or explicitly contradicted by the context in which it is used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, B and C together, or A and B and C together." In addition, use of the term "based on," above and in the claims is intended to mean, "based at least in part on," such that an unrecited feature or element is also permissible.

The subject matter described herein can be embodied in systems, apparatus, methods, and/or articles depending on the desired configuration. The implementations set forth in the foregoing description do not represent all implementations consistent with the subject matter described herein. Instead, they are merely some examples consistent with aspects related to the described subject matter. Although a few variations have been described in detail above, other modifications or additions are possible. In particular, further features and/or variations can be provided in addition to those set forth herein. For example, the implementations described above can be directed to various combinations and subcombinations of the disclosed features and/or combinations and subcombinations of several further features disclosed above. In addition, the logic flows depicted in the accompanying figures and/or described herein do not necessarily require the particular order shown, or sequential order, to achieve desirable results. Other implementations may be within the scope of the following claims.

**Claims**

1. A method for implementation by one or more programmable data processors (112, 508) forming part of at least one computing device (110), the method (400) comprising:

   continuously receiving (410) first data generated by a first physiological sensor (140) measuring at least one physiological parameter of a patient (130), wherein the at least one physiological parameter is cardiac output, wherein the first physiological sensor (140) is used to measure arterial pressure cardiac output;
   continuously receiving (420) second data generated by a second physiological sensor (150) concurrently measuring the at least one physiological parameter of the patient, wherein the second physiological sensor (150) is used to measure continuous cardiac output and/or injectate cardiac output,
   the first physiological sensor (140) measuring the at least one physiological parameter at a higher bandwidth with lower precision as compared to the second physiological sensor (150);
   adaptively calibrating (430) the continuously received first data using the continuously received second data to result in a continually updating calibrated measurement; and
   providing (440) data characterizing the continually updating calibrated measurement.

2. The method of claim 1, wherein the providing (440) data comprises one or more of: displaying the data characterizing the calibrated measurement in an electronic visual display (116), transmitting the data characterizing the calibrated measurement to a remote computing system (160, 170), loading the data characterizing the calibrated measurement into memory (114, 416), or storing the data characterizing the calibrated measurement in physical data persistence (552, 556, 560).

3. The method of claim 1, wherein the adaptive calibration is based on a time-varying linear scaling and an offset calculated using a least mean-square error solution.

4. The method of claim 3 further comprising: time averaging measurement values within the first data over a time window length corresponding to a periodicity of measurements of the second physiological sensor (150).

5. The method of claim 4 further comprising:
weighting the time averaged measurement values based on a standard deviation of the measurements from each of the first physiological sensor (140) and the second physiological sensor (150), and preferentially further comprising:

determining if a measurement value exceeds a pre-defined standard of deviation value; and
characterizing the measurement value as being a good measurement if it does not exceed the pre-defined standard of deviation value; or
characterizing the measurement value as being a bad measurement if it exceeds the pre-defined standard of deviation value.

6. The method of claim 4 further comprising: weighting the time averaged measurement values based on a forgetting factor.

7. A system comprising:

at least one programmable data processor (112, 508); and
memory (114, 416, 512) storing instructions which, when executed by the at least one programmable data processor, implement operations comprising:

continuously receiving first data generated by a first physiological sensor (140) measuring at least one physiological parameter of a patient, wherein the first physiological sensor (140) is used to measure arterial pressure cardiac output;
continuously receiving second data generated by a second physiological sensor (150) concurrently measuring the at least one physiological parameter of the patient, wherein the second physiological sensor (150) is used to measure continuous cardiac output and/or injectate cardiac output,
the first physiological sensor (140) measuring the at least one physiological parameter at a higher bandwidth with lower precision as compared to the second physiological sensor (150);
adaptively calibrating the continuously received first data using the continuously received second data to result in a continually updating calibrated measurement; and
providing data characterizing the continually updating calibrated measurement.

8. The system of claim 7 further comprising the first physiological sensor (140) and the second physiological sensor (150).

9. The system of claim 7, wherein the first physiological sensor (140) comprises a cuff to be placed on an extremity of the patient and utilizing a volume clamp method to calculate at least one physiological parameter selected from a group consisting of: stroke volume, stroke volume variation, APCO, systemic vascular resistance (SVR), or continuous blood pressure (cBP).

10. The system of claim 7, wherein the second physiological sensor (150) comprises a pulmonary artery catheter (PAC) that is inserted into a pulmonary artery of the patient to detect cardiac pressures in the patient by way of a thermal filament located on the catheter or the second physiological sensor (150) measures cardiac output using a bolus thermodilution method.

11. The system of claim 7, wherein the adaptive calibration is based on a time-varying linear scaling and an offset calculated using a least mean-square error solution.

12. The system of claim 11, wherein the operations further comprise:
time averaging measurement values within the first data over a time window length corresponding to a periodicity of measurements of the second physiological sensor, and wherein the operations preferentially further comprise: weighting the time averaged measurement values based on a forgetting factor.

13. The system of claim 11, wherein the operations further comprise:
weighting the time averaged measurement values based on a standard deviation of the measurements from each of the first physiological sensor (140) and the second physiological sensor (150).

14. The system of claim 12, wherein the operations further comprise:

determining if a measurement value exceeds a pre-defined standard of deviation value; and
characterizing the measurement value as being a good measurement if it does not exceed the pre-defined standard of deviation value; or
characterizing the measurement value as being a bad measurement if it exceeds the pre-defined standard of deviation value.


**Patentansprüche**

1. Verfahren zur Implementierung durch einen oder mehrere programmierbare Datenprozessoren (112, 508), die einen Teil mindestens einer Rechnervorrichtung (110) bilden, wobei das Verfahren (400) umfasst:

kontinuierliches Empfangen (410) erster Daten, die von einem ersten physiologischen Sensor (140) erzeugt werden, der mindestens einen physiologischen Parameter eines Patienten (130) misst, wobei der mindestens eine physiologische Parameter die Herzleistung ist, wobei der erste physiologische Sensor (140) verwendet wird, um eine Arteriendruck-Herzleistung zu messen;
kontinuierliches Empfangen (420) zweiter Daten, die von einem zweiten physiologischen Sensor (150) erzeugt werden, der gleichzeitig den mindestens einen physiologischen Parameter des Patienten misst, wobei der zweite physiologische Sensor (150) verwendet wird, um die kontinuierliche Herzleistung und/oder die Injektions-Herzleistung zu messen,
wobei der erste physiologische Sensor (140) den mindestens einen physiologischen Parameter mit einer höheren Bandbreite und einer geringeren Präzision im Vergleich zum zweiten physiologischen Sensor (150) misst;
adaptives Kalibrieren (430) der kontinuierlich empfangenen ersten Daten unter Verwendung der kontinuierlich empfangenen zweiten Daten, um zu einer kontinuierlich aktualisierten kalibrierten Messung zu gelangen; und
Bereitstellen (440) von Daten, die die kontinuierlich aktualisierte kalibrierte Messung kennzeichnen.

2. Verfahren nach Anspruch 1, wobei das Bereitstellen (440) von Daten eines oder mehrere umfasst von: Anzeigen der die kalibrierte Messung kennzeichnenden Daten in einer elektronischen visuellen Anzeige (116), Übertragen der die kalibrierte Messung kennzeichnenden Daten an ein entferntes Computersystem (160, 170), Laden der die kalibrierte Messung kennzeichnenden Daten in einen Speicher (114, 416) oder Speichern der die kalibrierte Messung kennzeichnenden Daten in einer physikalischen Datenpersistenz (552, 556, 560).

3. Verfahren nach Anspruch 1, wobei die adaptive Kalibrierung auf einer zeitvariablen linearen Skalierung und einem Offset basiert, die unter Verwendung einer Kleinstermittlerer-quadratischer-Fehler-Lösung berechnet werden.

4. Verfahren nach Anspruch 3, das ferner umfasst: zeitliche Mittelung der Messwerte innerhalb der ersten Daten über eine Zeitfensterlänge, die einer Periodizität der Messungen des zweiten physiologischen Sensors (150) entspricht.

5. Verfahren nach Anspruch 4, ferner umfassend:
Gewichten der zeitlich gemittelten Messwerte basierend auf einer Standardabweichung der Messungen von jeweils dem ersten physiologischen Sensor (140) und dem zweiten physiologischen Sensor (150), und vorzugsweise ferner umfassend:

Bestimmen, ob ein Messwert einen vordefinierten Standardabweichungswert überschreitet; und
Kennzeichnen des Messwerts als eine gute Messung, wenn er den vordefinierten Standardabweichungswert nicht überschreitet; oder

Kennzeichnen des Messwerts als eine schlechte Messung, wenn er den Standardabweichungswert überschreitet.

6. Verfahren nach Anspruch 4, das ferner umfasst: Gewichten der zeitlich gemittelten Messwerte basierend auf einem Vergessensfaktor.

7. System, umfassend:

mindestens einen programmierbaren Datenprozessor (112, 508); und
Speicher (114, 416, 512), der Befehle speichert, die dann, wenn sie von dem mindestens einen programmierbaren Datenprozessor ausgeführt werden, Operationen implementieren, die umfassen:

kontinuierliches Empfangen erster Daten, die von einem ersten physiologischen Sensor (140) erzeugt werden, der mindestens einen physiologischen Parameter eines Patienten misst, wobei der erste physiologische Sensor (140) verwendet wird, um eine Arteriendruck-Herzleistung zu messen;
kontinuierliches Empfangen von zweiten Daten, die von einem zweiten physiologischen Sensor (150) erzeugt werden, der gleichzeitig den mindestens einen physiologischen Parameter des Patienten misst, wobei der zweite physiologische Sensor (150) verwendet wird, um die kontinuierliche Herzleistung und/oder die Injektions-Herzleistung zu messen,
wobei der erste physiologische Sensor (140) den mindestens einen physiologischen Parameter mit einer höheren Bandbreite und einer geringeren Genauigkeit im Vergleich zum zweiten physiologischen Sensor (150) misst;
adaptives Kalibrieren der kontinuierlich empfangenen ersten Daten unter Verwendung der kontinuierlich empfangenen zweiten Daten, um eine kontinuierlich aktualisierte kalibrierte Messung zu erhalten; und
Bereitstellen von Daten, die die kontinuierlich aktualisierte kalibrierte Messung kennzeichnen.

8. System nach Anspruch 7, das ferner den ersten physiologischen Sensor (140) und den zweiten physiologischen Sensor (150) umfasst.

9. System nach Anspruch 7, wobei der erste physiologische Sensor (140) eine Manschette umfasst, die an einer Extremität des Patienten anzulegen ist und ein Volumen-Klammer-Verfahren verwendet, um mindestens einen physiologischen Parameter zu berechnen, der aus einer Gruppe ausgewählt ist, die umfasst: Schlagvolumen, Schlagvolumenvariation, APCO, systemischer Gefäßwiderstand (SVR), oder kontinuierlicher Blutdruck (cBP).

10. System nach Anspruch 7, wobei der zweite physiologische Sensor (150) einen Lungenarterienkatheter (PAC) umfasst, der in eine Lungenarterie des Patienten eingeführt wird, um die Herzdrücke in dem Patienten mittels eines auf dem Katheter befindlichen thermischen Filaments zu erfassen, oder der zweite physiologische Sensor (150) die Herzleistung unter Verwendung eines Bolus-Thermodilutionsverfahrens misst.

11. System nach Anspruch 7, wobei die adaptive Kalibrierung auf einer zeitvariablen linearen Skalierung und einem Offset basiert, die unter Verwendung einer Kleinstermittlerer-quadratischer-Fehler-Lösung berechnet werden.

12. System nach Anspruch 11, wobei die Operationen ferner umfassen:
zeitliches Mitteln von Messwerten innerhalb der ersten Daten über eine Zeitfensterlänge, die einer Periodizität von Messungen des zweiten physiologischen Sensors entspricht, und wobei die Operationen vorzugsweise ferner umfassen: Gewichten der zeitlich gemittelten Messwerte basierend auf einem Vergessensfaktor.

13. System nach Anspruch 11, wobei die Operationen ferner umfassen:
Gewichten der zeitlich gemittelten Messwerte basierend auf einer Standardabweichung der Messungen von jeweils dem ersten physiologischen Sensor (140) und dem zweiten physiologischen Sensor (150).

14. System nach Anspruch 12, wobei die Operationen ferner umfassen:

Bestimmen, ob ein Messwert einen vordefinierten Standardabweichungswert überschreitet; und
Kennzeichnen des Messwerts als eine gute Messung, wenn er den vordefinierten Standardabweichungswert nicht überschreitet; oder
Kennzeichnen des Messwerts als eine schlechte Messung, wenn er den vordefinierten Standardabweichungswert überschreitet.

**Revendications**

1. Procédé destiné à être mis en œuvre par un ou plusieurs processeurs (112, 508) programmables de données faisant partie d'au moins un dispositif informatique (110), le procédé (400) comprenant :

   la réception en continu (410) de premières données générées par un premier capteur physiologique (140) mesurant au moins un paramètre physiologique d'un patient (130), ledit au moins un paramètre physiologique étant un débit cardiaque, le premier capteur physiologique (140) étant utilisé pour mesurer un débit cardiaque sur la base de la pression artérielle ;

   la réception en continu (420) de secondes données générées par un second capteur physiologique (150) mesurant simultanément ledit au moins un paramètre physiologique du patient, le second capteur physiologique (150) étant utilisé pour mesurer un débit cardiaque continu et/ou un débit cardiaque injecté,

   le premier capteur physiologique (140) mesurant ledit au moins un paramètre physiologique à une largeur de bande supérieure avec une précision inférieure par rapport à celles du second capteur physiologique (150) ;

   l'étalonnage adaptatif (430) des premières données reçues en continu à l'aide des secondes données reçues en continu afin d'obtenir une mesure étalonnée continuellement mise à jour ; et

   la fourniture (440) de données caractérisant la mesure étalonnée continuellement mise à jour.

2. Procédé selon la revendication 1, la fourniture (440) de données comprenant un ou plusieurs éléments parmi : l'affichage des données caractérisant la mesure étalonnée dans un affichage visuel électronique (116), la transmission des données caractérisant la mesure étalonnée à un système informatique distant (160, 170), le chargement des données caractérisant la mesure étalonnée dans une mémoire (114, 416) ou le stockage des données caractérisant la mesure étalonnée dans une persistance (552, 556, 560) de données physiques.

3. Procédé selon la revendication 1, l'étalonnage adaptatif étant basé sur une mise à l'échelle linéaire variant dans le temps et sur un décalage calculé à l'aide d'une solution d'erreur quadratique moyenne minimale.

4. Procédé selon la revendication 3, comprenant en outre : le moyennage temporel de valeurs de mesure dans les premières données sur une longueur de fenêtre temporelle correspondant à une périodicité de mesures du second capteur physiologique (150).

5. Procédé selon la revendication 4, comprenant en outre :
   la pondération des valeurs de mesure moyennées dans le temps sur la base d'un écart-type des mesures provenant de chaque capteur parmi le premier capteur physiologique (140) et le second capteur physiologique (150) et comprenant de préférence en outre :

   la détermination du fait qu'une valeur de mesure dépasse ou non une norme prédéfinie de valeur d'écart ; et

   la caractérisation de la valeur de mesure comme étant une bonne mesure si elle ne dépasse pas la norme prédéfinie de valeur d'écart ; ou

   la caractérisation de la valeur de mesure comme étant une mauvaise mesure si elle dépasse la norme prédéfinie de valeur d'écart.

6. Procédé selon la revendication 4, comprenant en outre : la pondération des valeurs de mesure moyennées dans le temps sur la base d'un facteur d'oubli.

7. Système comprenant :

   au moins un processeur (112, 508) programmable de données ; et

   une mémoire (114, 416, 512) stockant des instructions qui, lorsqu'elles sont exécutées par ledit au moins un processeur programmable de données, mettent en œuvre des opérations comprenant :

   la réception en continu de premières données générées par un premier capteur physiologique (140) mesurant au moins un paramètre physiologique d'un patient, le premier capteur physiologique (140) étant utilisé pour mesurer un débit cardiaque sur la base de la pression artérielle ;

   la réception en continu de secondes données générées par un second capteur physiologique (150) mesurant simultanément ledit au moins un paramètre physiologique du patient, le second capteur physiologique (150) étant utilisé pour mesurer un débit cardiaque continu et/ou un débit cardiaque injecté,

   le premier capteur physiologique (140) mesurant ledit au moins un paramètre physiologique à une largeur de bande supérieure avec une précision inférieure par rapport à celles du second capteur physiologique (150) ; l'étalonnage adaptatif des premières données reçues en continu à l'aide des secondes données reçues en continu afin d'obtenir une mesure étalonnée continuellement mise à jour ; et la fourniture de données caractérisant la mesure étalonnée continuellement mise à jour.

**8.** Système selon la revendication 7, comprenant en outre le premier capteur physiologique (140) et le second capteur physiologique (150).

**9.** Système selon la revendication 7, le premier capteur physiologique (140) comprenant un brassard à placer sur une extrémité du patient et utilisant un procédé de serrage volumique afin de calculer au moins un paramètre physiologique choisi dans un groupe constitué par : un volume systolique, une variation de volume systolique, un APCO, une résistance vasculaire systémique (RVS) ou une pression artérielle continue (PAc).

**10.** Système selon la revendication 7, le second capteur physiologique (150) comprenant un cathéter d'artère pulmonaire (CAP) qui est inséré dans une artère pulmonaire du patient afin de détecter des pressions cardiaques chez le patient au moyen d'un filament thermique situé sur le cathéter ou le second capteur physiologique (150) mesurant un débit cardiaque à l'aide d'une méthode de thermodilution de bolus.

**11.** Système selon la revendication 7, l'étalonnage adaptatif étant basé sur une mise à l'échelle linéaire variant dans le temps et sur un décalage calculé à l'aide d'une solution d'erreur quadratique moyenne minimale.

**12.** Système selon la revendication 11, les opérations comprenant en outre : le moyennage temporel de valeurs de mesure dans les premières données sur une longueur de fenêtre temporelle correspondant à une périodicité de mesures du second capteur physiologique et les opérations comprenant en outre de préférence : la pondération des valeurs de mesure moyennées dans le temps sur la base d'un facteur d'oubli.

**13.** Système selon la revendication 11, les opérations comprenant en outre : la pondération des valeurs de mesure moyennées dans le temps sur la base d'un écart-type des mesures provenant de chaque capteur parmi le premier capteur physiologique (140) et le second capteur physiologique (150).

**14.** Système selon la revendication 12, les opérations comprenant en outre :

la détermination du fait qu'une valeur de mesure dépasse ou non une norme prédéfinie de valeur d'écart ; et la caractérisation de la valeur de mesure comme étant une bonne mesure si elle ne dépasse pas la norme prédéfinie de valeur d'écart ; ou

la caractérisation de la valeur de mesure comme étant une mauvaise mesure si elle dépasse la norme prédéfinie de valeur d'écart.

**FIG. 1**

EP 3 576 617 B1

**FIG. 2**

(a) Pig 309

**FIG. 3A**

(a) Pig 309

**FIG. 3B**

FIG. 4

EP 3 576 617 B1

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080287812 A1

- WO 0103580 A1